# EUROPEAN PATENT SPECIFICATION

(11) **EP 4 141 860 B1**
(45) Date of publication and mention of the grant of the patent: **09.07.2025**
(21) Application number: 21193489.8
(22) Date of filing: 27.08.2021
(51) Int. Cl.: G09G 5/02, G09G 3/20, G01N 21/27, G01N 21/29, G01N 21/78, G01N 21/84

(54) **METHODS AND DEVICES FOR CONTROLLING AUTO WHITE BALANCE SETTINGS OF A MOBILE DEVICE FOR A COLOR BASED MEASUREMENT USING A COLOR REFERENCE CARD**
METHODEN UND GERÄTE ZUR STEUERUNG DER EINSTELLUNGEN DES AUTOMATISCHEN WEISSABGLEICHS EINES MOBILGERÄTES FÜR EINE FARBBASIERTE MESSUNG MIT EINER FARBREFERENZKARTE
PROCÉDÉS ET DISPOSITIFS PERMETTANT DE CONTRÔLER LES RÉGLAGES AUTOMATIQUES DE BALANCE DES BLANCS D'UN APPAREIL MOBILE POUR UNE MESURE BASÉE SUR LES COULEURS Á L'AIDE D'UNE CARTE DE RÉFÉRENCE DES COULEURS

(43) Date of publication of application: 01.03.2023
(73) Proprietor: Roche Diabetes Care GmbH, 68305 Mannheim (DE); F. Hoffmann-La Roche AG, 4070 Basel (CH)
(72) Inventor: ALPEROWITZ, Lukas, 81241 Munich (DE); BERG, Max, 68305 Mannheim (DE); HAILER, Fredrik, 68305 Mannheim (DE); LIMBURG, Bernhard, 68305 Mannheim (DE)
(74) Representative: Kierig, Elisabeth

(56) References cited:
- EP-A2- 3 575 781
- US-A1- 2015 055 134

## Description

### Technical Field

The invention generally relates to a method of controlling auto white balance settings of a mobile device having a color camera and a plurality of preset white balance modes each associated with a different color temperature for performing a color based measurement using a color reference card comprising one or more gray fields each gray field having a known dedicated gray value. The invention further relates to a mobile device having at least one camera, to a kit for determining the concentration of the analyte in the sample of the bodily fluid, and further to a computer program and to a computer-readable storage medium. The methods, devices, computer program and storage medium specifically may be used in medical diagnostics, in order to for example qualitatively and/or quantitatively detect one or more analytes in one or more body fluids, such as for detecting glucose in blood and/or interstitial fluid. Other fields of application of the present invention, however, are also feasible.

### Background art

In the field of medical diagnostics, in many cases, one or more analytes have to be detected in samples of a body fluid, such as blood, interstitial fluid, urine, saliva or other types of body fluids. Examples of analytes to be detected are glucose, triglycerides, lactate, cholesterol or other types of analytes typically present in these body fluids. According to the concentration and/or the presence of the analyte, an appropriate treatment may be chosen, if necessary. Without narrowing the scope, the invention specifically may be described with respect to blood glucose measurements. It shall be noted, however, that the present invention may also be used for other types of analytical measurements using test strips.

Generally, devices and methods known to the skilled person make use of test strips comprising one or more test chemistries, which, in presence of the analyte to be detected, are capable of performing one or more detectable detection reactions, such as optically detectable detection reactions. With regard to these test chemistries, reference may be made e.g. to J. Hoenes et al.: The Technology Behind Glucose Meters: Test Strips, Diabetes Technology & Therapeutics, Volume 10, Supplement 1, 2008, S-10 to S-26. Other types of test chemistry are possible and may be used for performing the present invention.

Typically, one or more optically detectable changes in the test chemistry are monitored, in order to derive the concentration of the at least one analyte to be detected from these changes. For detecting the at least one change of optical properties of the test field of a test strip, various types of detectors (with various types of light sources for illuminating the test fields) are known (also referred to as dedicated meters or analytical measurement devices).

Further, besides using customized detectors, which are specifically developed for optically detecting changes in the test chemistry comprised by corresponding test strips, recent developments aim at using widely available devices such as smartphones. However, when consumer-electronics devices having a camera, such as smartphones, are employed instead of dedicated analytical measurement devices in order to determine analyte concentrations various influences need to be taken into account. As an example, lighting conditions, positioning, or other more or less uncontrollable conditions are to be considered.

To improve the reliability of a photometric analysis using a mobile device the EP 3 575 781 A2 discloses a specimen test strip to detect a characteristic of an analyte in a specimen sample with an additional color calibration area besides a reaction area for receiving the specimen sample.

A method of analyzing a colorimetric assay to identify a value for an assay parameter is disclosed by US 2015/0055134 A1 wherein intensity data for at least one of the color channels is converted to a first data point and compared with a standardized curve.

The US 2020/204718 A1 describes an auto white balance method wherein white balance settings are changed automatically to capture an aesthetically pleasing photograph, e.g. based on captured color values and/or a color emitted by a light source and/or on the subject of the photograph. Other auto white balance procedures are described in the US 9,628,704 B2 or the EP 2 498 498 B1.

WO 2019/056242 A1 describes a method to select a white balance mode for achieving certain aesthetic effects, especially when capturing images at night, i.e. with no or low ambient light.

WO 2021/010974 A1 describes an auto white balance procedure wherein an image is automatically segmented and different auto white balancing is applied to different segments.

US 2019/0007589 A1 describes an initialization method for a camera comprising different preset capture settings such as automatic white balance. For initialization the camera enters a recursive process of capturing one or more image frames, starting with default automatic white balancing settings, analyzing the image frames by measuring the color balance of at least a portion of the captured image frame, which may include a blue/green color ratio and/or a red/green color ratio, against one or more thresholds, and adjusting the automatic white balance settings based on the analysis. The capturing, measuring and adjusting is repeated until the color balance of the image capture falls within an acceptable range.

A basic white balancing method for a digital camera device using a reference sample is described in WO 2014/025415 A2. The method comprises identifying the reference sample that is white in standard lighting conditions in a captured image, determining the correction factor based at least partially on the average color of the white reference sample and applying the correction factor to the captured image and/or a further captured image.

Despite the advantages involved in using a mobile device and a color reference card for performing an analytical measurement, several technical challenges remain. Specifically, the use of mobile devices to determine analyte concentrations using test strips may require an accurate determination of the color change of the test strip and, thus, often remains challenging. Appropriate image brightness and color balance needs to be ensured. The observed brightness and light color in the captured images, e.g., may be dependent on various influencing factors, such as settings determining the exposure or the white balance when capturing the image as well as post-processing steps applied to the image after it was captured.

The determination of some settings such, as exposure settings or white balancing settings, by the mobile devices are often a device-specific process conducted in an automatic mode. Typically, however, automatic white balance of mobile devices, such as mobile phones or tablets, aim at producing aesthetically pleasing images and not at reproducing colors as authentically as possible. Specially, the auto white-balancing settings automatically selected by a mobile device may lead to over-saturation and/or clipping in one or more of the color channels, which will prevent the further processing and determination of a blood glucose value on the basis of the respective image.

### Problem to be solved

It is therefore desirable to provide methods and devices, which address the above-mentioned technical challenges of analytical measurements using mobile devices such as consumer-electronics mobile devices, specifically multipurpose mobile devices that are not dedicated to analytical measurements such as smartphones or tablet computers. Specifically, methods and devices shall be proposed which are widely applicable to available mobile devices and which are suited to increase measurement accuracy and convenience for the user.

### Summary

This problem is addressed by the methods and devices with the features of the independent claims. Advantageous embodiments, which might be realized in an isolated fashion or in any arbitrary combinations, are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, notwithstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect, a method of controlling auto white balance settings of a mobile device for performing a color based measurement using the mobile device and a color reference card is disclosed. The mobile device comprises a color camera and a plurality of preset white balance modes, each white balance mode associated with a different color temperature, and the color reference card comprises one or more gray fields each gray field having a known dedicated gray value.

The method comprises the following steps:
a. capturing using the camera and one of the white balance modes an image of at least part of the one or more gray fields of the color reference card,
   - the image comprising a plurality of color pixels and color information for at least three different colors for the plurality of pixels,
b. determining for a region of interest within the image a number of gray fields for which the color information for a first color shows overexposure as a first overexposure-number,
c. determining for the region of interest within the image a number of gray fields for which the color information for a second color shows overexposure as a second overexposure-number
   - wherein the first color relates to a smaller wavelength or a range of smaller wavelength than the second color,
d. re-capturing the image of step a) with a warmer white balance mode if one of the first and/or second overexposure-numbers or a sum of the first and second overexposure-numbers exceeds a respective preset overexposure-threshold, and the first overexposure number is greater than the second overexposure number,
e. re-capturing the image of step a) with a cooler white balance mode if one of the first and/or second overexposure-numbers or a sum of the first and second overexposure-numbers exceeds a respective preset overexposure-threshold, and the first overexposure number is smaller than the second overexposure number.

The method may comprise further steps, which are not listed.

The term "controlling" generally refers to a process of influencing or regulating settings or a process for determining settings in a defined or definable fashion.

The term "auto white balance settings" as used herein is a broad term, is to be given its ordinary and customary meaning to a person of ordinary skill in the art, and is not to be limited to a special or customized meaning. The term specifically may refer, without limitation, to an automated process aiming to adapt camera settings to prevailing conditions, especially to a scene lighting, so that neutral colors like gray or white in a scene also appear neutral in an image captured by the camera. The term may further refer, without limitation, to an algorithm for selecting preset white balance settings with a correct color temperature to fit the scene lighting.

The term "color temperature" of light, e.g. from a light source or ambient light, as used herein is a broad term, is to be given its ordinary and customary meaning to a person of ordinary skill in the art, and is not to be limited to a special or customized meaning. The term specifically may refer, without limitation, to the temperature of an ideal blackbody radiator that radiates light of a color comparable to the respective light.

The term "preset white balance mode" as used herein is a broad term, is to be given its ordinary and customary meaning to a person of ordinary skill in the art, and is not to be limited to a special or customized meaning. The term specifically may refer to a set of camera parameters for color balancing or chromatic adaptation to a specific color temperature, i.e. a specific light condition such as daylight or flashlight or other artificial or natural lighting. The camera parameters may relate, without limitation, to a scaling of the relative luminance by introducing relative scaling factors for different color channels of the camera. Examples for typical preset white balance modes, without limitation, are of the categories automatic, daylight, incandescent light, fluorescent light, cloudy, speed light, i.e. flash, and/or white balance preset.

The term "mobile device" as used herein is a broad term, is to be given its ordinary and customary meaning to a person of ordinary skill in the art, and is not to be limited to a special or customized meaning. The term specifically may refer, without limitation, to a mobile electronics device, more specifically to a mobile communication device such as a cell phone or a smartphone. Additionally or alternatively, as will be outlined in further detail below, the mobile device may also refer to a tablet computer or another type of portable computer having at least one color camera.

The term "color reference card" as used herein is a broad term, is to be given its ordinary and customary meaning to a person of ordinary skill in the art, and is not to be limited to a special or customized meaning. The term specifically may refer, without limitation, to an arbitrary item having, disposed therein or disposed thereon, such as on at least one surface, one or more gray fields having defined gray levels, i.e. gray values, and further, for example, a plurality of different color reference fields having known color properties or optical properties, such as having a plurality of colored fields having known reference color values. As an example, the color reference card may be a flat card comprising at least one substrate having, on at least one surface and/or disposed therein, the plurality of color reference fields having known color coordinates and one or more gray fields having known gray levels. The color reference card may also comprise one or more further gray fields. This will be described further below.

The substrate, specifically, may have a flat surface comprising one or more gray fields, and e.g. additionally a plurality of color reference fields and/or further gray fields. The substrate, as an example, may be or may comprise one or more of a paper substrate, a cardboard substrate, a plastic substrate, a ceramic substrate or a metal substrate. Laminate substrates are also possible. The substrate, as an example, may be sheet-like or flexible. It shall be noted, however, that the substrate may also be implemented into an article of use, such as into a wall of a box, a vile, a container, a medical consumable, such as a test strip, or the like. Thus, the color reference card may also fully or partially be integrated into a test strip. Thus, the at least one image of at least a part of the color reference card may fully or partially comprise an image of at least part of the test strip having at least one test field.

Further, the color reference card may comprise at least one position marker. The at least one position marker, as an example, may be or may comprise particularly an ArUco code or the like. Specifically, the at least one position marker may be arranged in at least one corner of the color reference card. Thus, the mobile device may be configured for detecting and/or reading the marker, specifically by optically detecting the marker (e.g., on the at least one image captured in step a)), and optionally retrieving information from the marker, such as information on the position and/or orientation of the color reference card in relation to the camera. The color reference card may include other marker(s) including further information or the position marker may additionally include further information. Such further information may include at least one of: an identifier for identifying the color reference card and/or the type of the color reference card, such as at least one of a label, a barcode or a QR-code; a specifier specifying details of the color reference card, such as reference color values, gray values and/or further gray background values or the like, such as by using at least one of a label, a barcode or a QR-code.

The term "gray field" as used herein is a broad term, is to be given its ordinary and customary meaning to a person of ordinary skill in the art, and is not to be limited to a special or customized meaning. The term specifically may refer, without limitation, to an arbitrary item having known optical properties, such as a known reference color value. Specifically, a gray field comprised by the color reference card may be a 2-dimensional structure, such as a rectangle, a square, a polygon, a circle and/or an ellipse, with a uniform gray value, i.e. a dedicated gray value. The gray value of the gray field specifically may be one or more of predetermined, known or determinable. For example, gray values result when for r=g=b, i.e. a red, green and blue color value or color channel for an image point/pixel have equal values. The gray field may be comprised by a surface of the color reference card and/or disposed therein, specifically in such a way that at least part of the one or more gray fields may be visible in the image captured in step a).

The gray fields of the color reference card may be arranged in a regular pattern on the surface of the color reference card, such as in a rectangular pattern, e.g. a rectangular matrix pattern. The pattern arrangement specifically may enable identifying the gray fields, such as by searching at a predetermined distance in an x- and/or y-direction from one or more of the position markers.

Further, the one or more gray fields may be locally distributed over the color reference card, specifically over a part of the color reference card visible in the image.

The term "color camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. The camera may specifically comprise one or more imaging devices, such as camera chips or imaging chips, e.g. CCD and/or CMOS chips. The color camera, in particular the imaging device, may comprise a one-dimensional or two-dimensional array of image sensors, such as pixels. As an example, the color camera may comprise at least 10 pixels in at least one dimension, such as at least 10 pixels in each dimension. It shall be noted, however, that other cameras are also feasible. The color camera, besides at least one camera chip or imaging chip, may comprise further elements, such as one or more optical elements, e.g. one or more lenses. As an example, the camera may be a fix-focus camera, having at least one lens, which is fixedly adjusted with respect to the camera. Alternatively, however, the camera may also comprise one or more variable lenses that may be adjusted, automatically or manually. The camera may also comprise a diaphragm for controlling the aperture (whereby the amount of light that reaches the camera sensor may be controlled). The diaphragm functions much like the iris of the eye- it controls the effective diameter of the lens opening (called the aperture). The camera may further comprise an ambient light sensor for measuring light reflected by a scene to be captured in an image.

The invention specifically shall be applicable to cameras as usually used in mobile applications such as notebook computers, tablets or, specifically, cell phones such as smartphones. Thus, specifically, the color camera may be part of a mobile device which, besides the at least one camera, comprises one or more data processing devices such as one or more data processors. Other color cameras, however, are feasible.

For each pixel of the color camera, color information may be provided or generated, such as color values for three colors R, G, B. A larger number of color values is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. For example, gain can be a digital camera setting that controls the amplification of the signal from the camera sensor.

The term "image" as used herein is a broad term, is to be given its ordinary and customary meaning to a person of ordinary skill in the art, and is not to be limited to a special or customized meaning. The term may specifically refer, without limitation, to a set of spatially resolved optical data. Specifically, the term may relate to data recorded by using a camera, such as a plurality of electronic readings from the imaging device, such as the pixels of the camera chip. Further, the term may refer, without limitation, to a color digital image made of pixels, each pixel comprising color information for at least three different colors, i.e. at least three different wavelengths of light. For example, each pixel is made of a combination of color values, especially for different primary colors such as red, green and blue. In an embodiment the image may comprise a plurality of color pixels, each color pixel comprising an n-tuple of at least three color values for three different colors. In an alternative embodiment, the image may further comprise at least three different color channels, wherein a color channel may refer to a grayscale image, of the same size as the image, made of just one of the at least three colors.

The term "capturing an image" as used herein is a broad term, is to be given its ordinary and customary meaning to a person of ordinary skill in the art and is not to be limited to a special or customized meaning. The term specifically may refer, without limitation, to one or more of imaging, image recording, image acquisition, image capturing. The term "capturing an image" may comprise capturing at least one single image, i.e. one image and/or a plurality of images such as a sequence of images. For example, the capturing of the image may comprise recording continuously a sequence of images such as a video or a movie. The capturing of the image may be initiated by user action or may automatically be initiated, e.g. once the presence of the at least one object within a field of view and/or within a predetermined part of the field of view of the camera is automatically detected. These automatic image acquisition techniques are known e.g. in the field of automatic barcode readers, such as from automatic barcode reading apps. The capturing of the images may take place, as an example, by acquiring a stream or "live stream" of images with the camera, wherein one or more of the images, automatically or by user interaction such as pushing a button, are stored and one of them is used as the at least one image. The image acquisition may be supported by a processor of the mobile device, and the storing of the images may take place in a data storage device of the mobile device.

The capturing of the image of at least part of the one or more gray fields of the color reference card by using the color camera may imply taking an image which at least comprises a region of interest within the at least one gray field. Thus, as an example, one or more gray fields may be detected automatically within the image, e.g. by pattern recognition techniques generally known to the skilled person, and at least one region of interest may be chosen within each gray field, e.g. a rectangular, square, polygonal, oval or round region of interest. The taking of the image may be initiated by the user action or may automatically be initiated once the presence of the at least one gray field within a field of view and/or within a predetermined sector of the field of view of the camera is automatically detected.

The term "region of interest" as used herein is a broad term, is to be given its ordinary and customary meaning to a person of ordinary skill in the art, and is not to be limited to a special or customized meaning. The term specifically may refer, without limitation, to a selected portion of the image, said portion comprising one or more areas within the image or the whole image. The region of interest may be determined by selecting an area within the image, e.g. automatically determining and selecting an area within the image comprising at least one gray field. The area or portion of the image may refer to a corresponding part of the pixels, e.g. color pixels, of the image and/or a corresponding part of each of the color channels of the image.

The terms "overexposed" and "overexposure" as used herein are a broad term. The terms specifically may refer, without limitation, to an amount of light per unit area or the amount of light of a certain wavelength or range of wavelength exceeding a preset threshold or exceeding the amount that can be reliably captured, e.g. when the intensity in an image area falls outside the maximum intensity, which can be represented. Alternatively and without limitation, the term may refer to a guide value, such as an average value or a median or a maximum, determined for the region of interest said guide value exceeding a preset threshold.

Thus the "determining for a region of interest within the image a number of gray fields for which the color information for a certain color shows overexposure" may refer without limitation to determining for the region of interest within the image a number of gray fields for which the color information for a certain color exceeds a preset threshold.

"Overexposure of the color information for a certain color for the region of interest" may refer, without limitation, to the color information for the region of interest exceeding a preset threshold or corresponding to a maximum. Alternatively and without limitation, it may refer to one, more or all pixels within the region of interest exceeding a preset threshold or corresponding to a preset maximum value. In another alternative, without limitation, it may refer to a guide value exceeding a preset threshold or corresponding to a preset maximum, the guide value being determined for the region of interest. The guide value, without limitation, may refer to an average, a median or a maximum determined for the respective pixels. Furthermore, the guide value, without limitation, may be determined on the bases of one, more or all pixels of the region of interest.

According to steps d) and e) the method jumps back to step a) and a new image is captured, i.e. the image is re-captured, if the first and/or second overexposure-number or a sum of the first and second overexposure-number exceeds a preset respective overexposure-threshold. This may imply, that either one overexposure-number or both overexposure-numbers or the sum or a combination of the aforementioned values is compared to a preset appropriate threshold and that a re-capturing is performed if one or if all of the values compared to a threshold exceed the respective threshold. The value of either of the overexposure-numbers or a sum of both relates to a degree of overexposure in the image.

For the re-capturing of the image, a new white balance mode is selected based on an analysis of the determined overexposure-numbers. If the first overexposure-number is greater than the second overexposure-number, i.e. the overexposure for light with a smaller wavelength, i.e. cooler color temperature, exceeds the overexposure for greater, warmer wavelengths a white balance mode associated with a warmer color temperature, i.e. a warmer white balance mode is selected. Correspondingly, if a greater overexposure is determined for greater, warmer wavelength, i.e. the second color, a white balance mode associated with a cooler color temperature (cooler white balance mode) is selected. For example, the next cooler or warmer white balance mode is selected, respectively. Alternatively, any one of the cooler or warmer white balance modes is selected. According to an embodiment, a difference between the two overexposure-numbers is determined and the warmer or cooler white balance mode is selected on the basis of the determined difference.

An advantage of the described method is, that on the bases of the preset device conditions, i.e. the preset white balance modes available for the device, the best mode is selected in a directed recursive process. Therefore, the described method is particularly easy and fast and helps to increase the reliability of a color measurement via a mobile device.

In the described recursive process the preset white balance modes may be selected following preset order, e.g. the next warmer or colder mode is selected or the next but one warmer or colder mode is selected or any one of the warmer or colder modes is selected.

Furthermore, it is understood that the recursive process may end at any reasonable break point. A break condition may, for example, be the fact that all preset white balance modes have been used or that the first and second overexposure-number are equal or that the difference between the first and second overexposure-number has reached a minimum or that said difference starts growing again.

According to an embodiment, the method steps b) and c) comprise identifying at least a portion of the color information for the respective color corresponding to the gray fields within the region of interest, determining a guide value for the identified portion of the color information and determining whether the guide value exceeds a predetermined overexposure-threshold.

The identifying may refer to finding all or a portion, e.g. based on predefined conditions, of the pixels and/or color information corresponding to the gray fields. For example, a plurality of color pixels or of pixels of a color channel and the corresponding respective values for the color are identified.

The term "guide value" as used herein is a broad term, is to be given its ordinary and customary meaning to a person of ordinary skill in the art and is not to be limited to a special or customized meaning. The term specifically may refer, without limitation, to an average value, a median, and/or a maximum value determined for the identified color information, e.g. the plurality of values for the specific color of the identified color pixels or the plurality values of the identified pixels of the specific color channel.

According to another embodiment, the second color is red and the first color is blue or green. Preferably, the first color is blue as in this case the difference between the corresponding wavelength of the first and second color is bigger than for the first color being green.

According to a further embodiment, the color based measurement is an analyte measurement based on a color formation reaction, wherein the color formation reaction occurs in a test strip having a sample applied to a test field of the test strip.

The term "color formation reaction" as used herein is a broad term, is to be given its ordinary and customary meaning to a person of ordinary skill in the art, and is not to be limited to a special or customized meaning. The term specifically may refer, without limitation, to a chemical, biological or physical reaction during which a color, specifically a reflectance, of at least one element involved in the reaction, changes with the progress of the reaction. Thus, as an example, reference may be made to the above-mentioned biochemical reactions, which typically are used for detecting blood glucose, involving a color change. Other types of color changing or color formation reactions are known to the skilled person, such as typical chemical reactions for determining the pH value.

The term "test strip" as used herein is a broad term, is to be given its ordinary and customary meaning to a person of ordinary skill in the art, and is not to be limited to a special or customized meaning. The term specifically may refer, without limitation, to a strip shaped element or device configured for performing a color-change detection reaction. The test strip may also just be referred to as test strip herein, wherein these terms may refer to the same element. The test strip may particularly have a test field, e.g. containing at least one test chemical for detecting at least one analyte. The test strip, as an example, may comprise at least one substrate, such as at least one carrier, with the at least one test field applied thereto or integrated therein. In particular, the test strip may further comprise at least one white area, such as a white field, specifically in a proximity to the test field, for example enclosing or surrounding the test field. The white area may be a separate field independently arranged on the substrate or carrier. However, additionally or alternatively, the substrate or carrier itself may be or may comprise the white area. The at least one carrier may be strip-shaped, thereby providing the basic form of the test strip. These test strips are generally widely in use and available. One test strip may carry a single test field or a plurality of test fields having identical or different test chemicals comprised therein.

As further used herein, the term "test field" is a broad term, is to be given its ordinary and customary meaning to a person of ordinary skill in the art, and is not to be limited to a special or customized meaning. The term specifically may refer, without limitation, to a coherent amount of the test chemical, such as to a field or a region, e.g. a field of round, polygonal or rectangular shape, having one or more layers of material, with at least one layer of the test field having the test chemical comprised therein.

The test strip may be placed on top of the color reference card, and/or the color reference card may comprise one or more windows, wherein the color reference card, with the one or more windows, is placed on top of the test strip such that the test field or at least a part of the test field is visible through the window.

According to another embodiment, the color reference card further comprises a plurality of different color reference fields having known reference color values.

The term "color reference field" as used herein is a broad term, is to be given its ordinary and customary meaning to a person of ordinary skill in the art and is not to be limited to a special or customized meaning. The term specifically may refer, without limitation, to an arbitrary item having known optical properties, such as a known reference color value. Specifically, a color reference field comprised by the color reference card may be a 2-dimensional structure, such as a rectangle, a square, a polygon, a circle and/or an ellipse, with a uniform color value. The color value of the color reference field specifically may be one or more of predetermined, known or determinable. The color reference field may be comprised by a surface of the color reference card and/or disposed therein, specifically in such a way that at least part of the plurality of different color reference fields (e.g. one color reference field) may be visible in the image captured in step c).

Further, the color reference fields may have color values in a subspace of the color coordinate system corresponding to the color space of the color formation reaction of the reagent test region. The color reference fields of the color reference card specifically may be arranged in a regular pattern on the surface of the color reference card, such as in a rectangular pattern, e.g. a rectangular matrix pattern. The pattern arrangement specifically may enable identifying the color reference fields, such as by searching at a predetermined distance in an x- and/or y-direction from one or more of the position markers.

According to a further embodiment, the image captured in step a) additionally captures at least part of the test field of the test strip. This helps to ensure that the captured image may be used to perform a color based measurement, e.g. to determine an analyte concentration. Therewith, a further step of capturing an image for the color-based measurement is not necessary.

According to another embodiment, the method further comprises determining a concentration of the analyte in the sample by using the image captured in step a), if a sum of the first overexposure-number and the second overexposure-number is smaller than the preset overall overexposure-threshold and/or if a difference between the first overexposure-number and the second overexposure-number is smaller than a preset balance threshold. Alternatively, a further image is captured and used for determining a concentration of the analyte in the sample if the aforementioned conditions are fulfilled.

According to a further embodiment, steps a) to e) are iterated until a difference between the first overexposure-number and the second overexposure-number is smaller than a preset balance threshold, or until step a) has been performed for all white balancing modes of the plurality of white balancing modes, or until a difference between the first overexposure-number and the second overexposure-number starts increasing compared to a difference determined based on a previously captured image, and then a concentration of the analyte in the sample is determined using the image corresponding to the smallest difference between the first overexposure-number and the second overexposure-number, or using an extra image captured using the auto white balancing mode corresponding to the smallest difference between the first overexposure-number and the second overexposure-number.

According to another embodiment, the mobile device comprises a display, and the method further comprises the step of providing visual guidance on the display for positioning the camera relative to a scene, the scene comprising at least part of the test strip and/or at least part of the color reference card. The visual guidance, e.g., comprises one or more arrows, frames or lines indicating a preferred positioning of the camera relative to the object. As one example, the visual guidance comprises an outline, which corresponds to the shape of the object superimposed on the display of the mobile device.

Providing visual guidance on the display for positioning the camera relative to an object on the display for determining the exposure parameters and/or for capturing the at least one image may in particular relate to providing visual guidance on the display in order to guide the user to position the camera such that the scene comprises at least part of the one or more gray fields of the color reference card. The guidance may additionally provide visual guidance to guide the user to position the camera such that the scene additionally comprises further structures such as at least part of the test field of the test strip having the sample applied thereto and/or at least part of the plurality of different color reference fields of the color reference card. The visual guidance, e.g., may comprise an outline, which corresponds to the shape of at least part of the color reference card and/or at least part of the test strip superimposed on the display of the mobile device.

According to a further embodiment, the color reference card comprises at least one position marker.

According to another embodiment the method is initiated automatically in case it is determined that the camera is in a defined position with respect to the color reference card based on the at least one position marker.

According to a further embodiment, the test field is in a defined position with respect to the color reference card during capturing the at least one image.

According to a further aspect of the invention a mobile device is disclosed having at least one camera and at least one display, the mobile device being configured for performing the method as described above.

According to another aspect of the invention a kit for determining the concentration of an analyte in a bodily fluid is disclosed, the kit comprising the above-described mobile device, at least one test strip having at least one test field, at least one color reference card, wherein the color reference card comprises a plurality of different color reference fields having known reference color values and one or more gray fields having a defined gray value.

According to a further aspect of the invention a computer program comprising instructions which, when the program is executed by a mobile device having a camera, cause the mobile device to carry out at least steps a) to d) of the methods as described above.

According to another aspect of the invention a computer-readable storage medium is disclosed, specifically a non-transitory storage medium, comprising instructions which, when executed by a mobile device having a camera, cause the mobile device to carry out at least steps a) to e) of the method as described above.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows a color reference card in top view;
- Figure 2: shows an embodiment of a kit comprising the mobile device, a color reference card and a test strip;
- Figure 3: shows the situation when capturing an image of the color reference card; and
- Figure 4: shows a flow chart of an embodiment of a method of controlling white balance settings.

### Detailed description of the embodiments

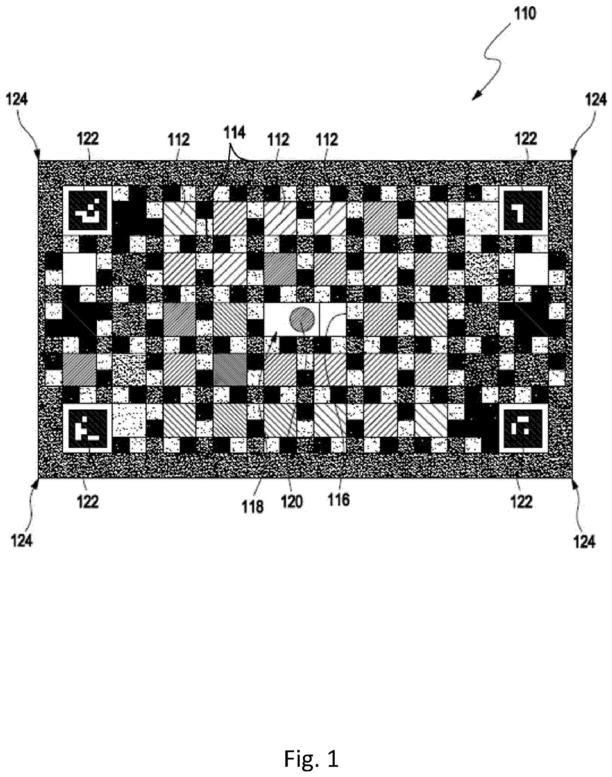

In Figure 1, an exemplary embodiment of a color reference card 110 is shown in a plan view. The color reference card 110 comprises one or more gray fields 114 having known gray values as well as a plurality of color reference fields 112 having known reference color values. The gray fields 114 and the color reference fields 112 may be arranged on the surface of the color reference card 110, such as on a substrate of the color reference card 110.

The color reference fields 112 may be distributed equally over the surface of the color reference card 110, specifically in such a way that the plurality of color reference fields 112may be distributed over the entire surface of the color reference card 110. As an example, the color reference fields 112 may be arranged in matrix pattern, such as a rectangular matrix pattern. However, the color reference fields 112 may also be arranged in other ways.

The one or more gray fields 114 may be surrounding the color reference fields 112 and/or framing the color reference card and/or be distributed along rows and/or columns between the color reference fields 112 matrix pattern. The color reference fields 112 and the gray fields 114 may not overlap each other.

The color reference card 110 may further comprise at least one window 116. Thus, at least one test strip 118 or a part thereof may be visible through the window 116 when the color reference card 110 is placed on top of the test strip 118. Specifically, at least one test field 120 comprised by the test strip 118 may be visible through the window 116 of the color reference card 110. As another example, the color reference card 110 may comprise the test strip 118 having at least one test field 120, specifically in such a way that the at least one test field 120 is accessible and visible.

Further, the color reference card 110 may comprise at least one position marker 122. The position marker 122 may particularly be an ArUco code. In Figure 1, the position marker 122 specifically may comprise one or more ArUco codes, such as in the corners of a rectangular matrix comprising the color reference fields 112 and some of the gray field 114 and being surrounded by a gray field 114. Thus, generally, the position marker 122 may be arranged in at least one corner 124 of the color reference card 110. For example, at least one position marker 122 may be arranged in each of the corners 124 of the color reference card 110, specifically in such a way that the position marker 122 may be visible together with the plurality of color reference fields 112. Further, the position marker 122 may comprise information about the orientation of the color reference card 110.

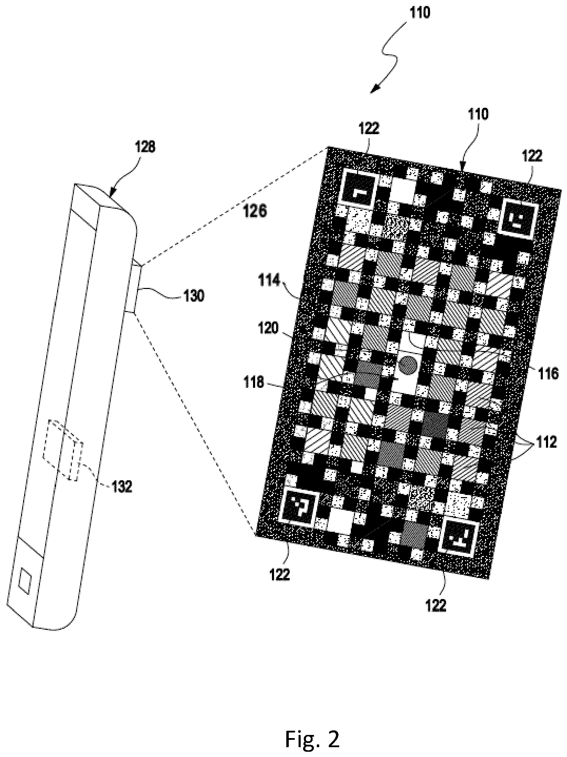

In Figure 2, an exemplary embodiment of a kit is shown in a perspective view. The kit comprises at least one mobile device 128 and at least one color reference card 110. Further, the kit comprises the at least one test strip 118.

The mobile device 128 may be or may comprise at least one of a cell phone, a smartphone, a tablet computer or the like. Further, the mobile device 128 has at least one color camera 130. The color camera 130 of the mobile device 128 may be configured for recording images, specifically color images. For example, the color camera 130 comprises at least three color sensor types, such as at least one color sensor type for red, green and blue, respectively.

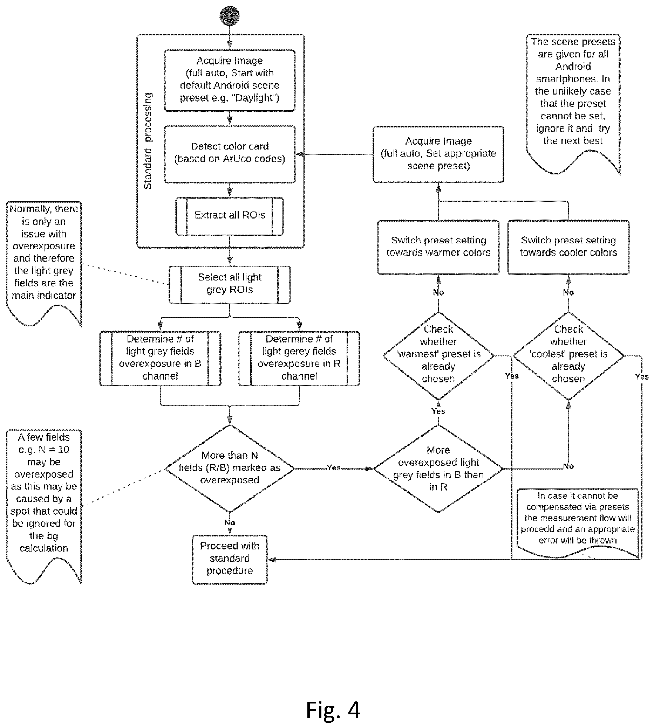

Further, the mobile device 128 may comprise at least one processor 132. The processor132 may be configured, specifically by software programming, to perform one or more of the method steps a) to d) of the method of controlling auto white balance settings. Exemplary embodiments of the above-mentioned methods are shown in Figures 4 and will be described in further detail below. Thus, reference may be made to the description of Figures 4.

The processor 132 may specifically be configured for supporting the setting of a region of interest and determining a first and second overexposure-number for the gray fields in the region of interest. The processor 132 may further specifically be configured for capturing at least one image comprising a scene 126. In the embodiment shown, the scene 126 comprises the entire color reference card 110 and the test field 120 of the test strip 118. Specifically, the processor 132 may prompt a user of the mobile device 128 to capture the image. Additionally or alternatively, the processor 132 may be configured for automatically capturing the image of the color reference card 110, specifically when the color reference card 110 may be in a field of view of the camera 130.

The color reference card 110 has been described above (see Figure 1).

The at least one marker 122 of the color reference card 110 may be used for identifying the color reference card 110. Specifically, the processor 132 of the mobile device 128 may be configured for detecting the position marker 122 in a field of view of the color camera 130.

The color reference card 110 and the test strip 118, may be visible on the at least one image captured by the color camera 130 of the mobile device 128. Specifically, the at least one test region 120 of the test strip 118 may be visible through the window 116 of the color reference card 110.

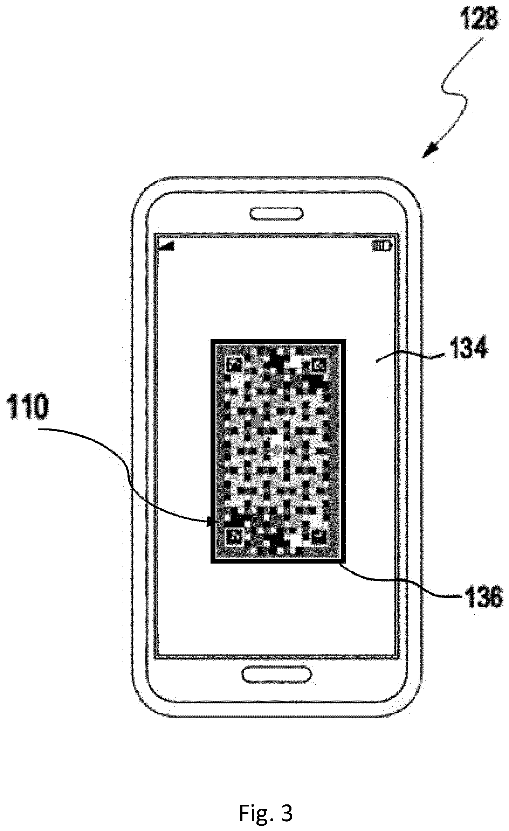

Figure 3 shows the situation when capturing the image. Visual guidance is provided in this embodiment on the display in form of an outline 136 of the color reference card superimposed on the display 134 of the mobile device 128 in order to guide the user to position the camera such that the captured scene 126 which corresponds to the outline 136 comprises at least part of one or more gray fields. In the example shown, the scene in the set exposure metering area comprises the entire color reference card.

Controlling the auto white balance settings and capturing the image may be initiated automatically. The capturing of an image may, e.g., be initiated automatically in case it is detected that, based on the position marker 122, the color reference card is within field of view of the camera 130.

Figure 4 shows a flow chart of an exemplary embodiment of a method of controlling auto-exposure settings of a mobile device 128.

The method is executed by the processor and/or other processing means and starts with acquiring using the camera 130 and one of the white balance modes an image of at least part of the one or more gray fields 114 of the color reference card 110, the image comprising a plurality of pixels and color information for at least three different colors for the plurality of pixels, and wherein in the shown embodiment the color reference card is detected in the image based on the ArUco codes;

For at least one region of interest ROI within the image a number of gray fields 114 for which the color information for a first color, channel B, shows overexposure is determined as a first overexposure-number. Correspondingly, for the same regions of interest ROI within the image a number of gray fields 114 for which the color information for a second color, channel R, shows overexposure is determined as a second overexposure-number, wherein the first color, here blue, relates to a smaller wavelength or a range of smaller wavelength than the second color, here red;

It is determined whether the sum of the first and second overexposure-numbers exceeds a threshold N and in case of no exaggerated overexposure, i.e. the sum of first and second overexposure-numbers not exceeding the threshold N, the color based measurement is performed with current image or a newly captured image using the current white balance mode.

In case of an exaggerated overexposure, i.e. the sum of first and second overexposure-numbers exceeding the threshold N, the first overexposure-number for channel B and the second overexposure-number for channel R are compared.

If the first overexposure-number for B is larger than the second overexposure-number for R in the shown embodiment it is checked whether the used white balance mode already has been the warmest and if this is the case the color based measurement is performed with current image or a newly captured image using the current white balance mode. If the used white balance mode is not the warmest a white balance mode associated with a warmer color temperature is selected from the plurality of white balance modes and the described process is started again acquiring an image using the newly selected white balance mode.

If the first overexposure-number for B is not larger than the second overexposure-number for R in the shown embodiment it is checked whether the used white balance mode already has been the coolest and if this is the case the color based measurement is performed with current image or a newly captured image using the current white balance mode. If the used white balance mode is not the coolest a white balance mode associated with a cooler color temperature is selected from the plurality of white balance modes and the described process is started again acquiring an image using the newly selected white balance mode.

The method may comprise additional steps that are not described above.

### List of reference numbers

- 110: color reference card
- 112: color reference field
- 114: gray field
- 116: window
- 118: test strip
- 120: test field
- 122: position marker
- 124: corner
- 126: scene
- 128: mobile device
- 130: camera
- 132: processor
- 134: display
- 136: outline

## Claims

1. A method of controlling auto white balance settings of a mobile device (128) for performing a color based measurement using the mobile device (128) and a color reference card (110),
- wherein the mobile device (128) comprises at least one processor (132), a color camera (130) and a plurality of preset white balance modes, each white balance mode associated with a different color temperature, and
- wherein the color reference card (110) comprises gray fields (114) each gray field having a known dedicated gray value,
the method comprising:
a. Capturing, supported by the at least one processor (132), using the camera (130) and one of the white balance modes an image of at least part of the gray fields (114) of the color reference card (110),
i. the image comprising a plurality of pixels and color information for at least three different colors for the plurality of pixels, each color corresponding to a wavelength of light,
**characterized in that** the method further comprises:
b. determining, by the processor (132), for a region of interest within the image a number of gray fields (114) for which the color information for a first color shows overexposure by exceeding a preset threshold as a first overexposure-number,
c. determining, by the processor (132), for the region of interest within the image a number of gray fields (114) for which the color information for a second color shows overexposure by exceeding a preset threshold as a second overexposure-number
i. wherein the first color relates to a smaller wavelength or a range of smaller wavelength than the second color,
d. re-capturing, supported by the processor (132), the image of step a) with a white balance mode associated with a warmer color temperature if one of the first and second overexposure-numbers or a sum of the first and second overexposure-numbers exceeds a respective preset overexposure-threshold, and the first overexposure number is greater than the second overexposure number,
e. re-capturing, supported by the processor (132), the image of step a) with a white balance mode associated with a cooler color temperature if one of the first and second overexposure-numbers or a sum of the first and second overexposure-numbers exceeds a respective preset overexposure-threshold, and the first overexposure number is smaller than the second overexposure number.

2. The method according to claim 1, wherein steps b) and c) comprise:
a. identifying at least a portion of the color information for the respective color corresponding to the gray fields within the region of interest,
b. determining a guide value for the identified portion of the color information
c. determining whether the guide value exceeds a predetermined overexposure-threshold.

3. The method according to claim 1 or 2, wherein the second color is red and the first color is blue or green.

4. The method according to any of the preceding claims, wherein the color based measurement is an analyte measurement based on a color formation reaction, wherein the color formation reaction occurs in an optical test strip (118) having a sample applied to a test field (120) of the test strip (118).

5. The method according to any of the preceding claims, wherein the color reference card (110) further comprises a plurality of different color reference fields (112) having known reference color values.

6. The method according to any of the preceding claims, wherein the image captured in step a) additionally captures at least part of the test field (120) of the optical test strip (118).

7. The method according to any of the preceding claims, wherein the method further comprises:
a. determining a concentration of the analyte in the sample by using the image captured in step a),
i. if a sum of the first overexposure-number and the second overexposure-number is smaller than the preset overall overexposure-threshold and/or
ii. if a difference between the first overexposure-number and the second overexposure-number is smaller than a preset balance threshold.

8. The method according to any of the preceding claims, wherein steps a) to e) are iterated
a. until a difference between the first overexposure-number and the second overexposure-number is smaller than a preset balance threshold, or
b. until step a) has been performed for all white balancing modes of the plurality of white balancing modes, or
c. until a difference between the first overexposure-number and the second overexposure-number starts increasing compared to a difference determined based on a previously captured image,
and then a concentration of the analyte in the sample is determined
d. using the image corresponding to the smallest difference between the first overexposure-number and the second overexposure-number, or
e. using an extra image captured using the auto white balancing mode corresponding to the smallest difference between the first overexposure-number and the second overexposure-number.

9. The method according to any of the preceding claims, wherein the mobile device (128) comprises a display (134), and the method further comprises the step of:
a. providing visual guidance on the display (134) for positioning the camera (130) relative to a scene, the scene comprising at least part of the optical test strip (118) and/or at least part of the color reference card (110).

10. The method according to any of the preceding claims, wherein the color reference card comprises at least one position marker (122).

11. The method according to claim 10, wherein method is initiated automatically in case it is determined that the camera (130) is in a defined position with respect to the color reference card (110) based on the at least one position marker (122).

12. A mobile device (128) having at least one camera (130) and at least one display (134), the mobile device (128) being configured for performing the method according to any of the preceding claims.

13. A kit for determining the concentration of an analyte in a bodily fluid, the kit comprising:
a. the mobile device according to claim 12,
b. at least one optical test strip (118) having at least one reagent test field (120),
c. at least one color reference card (110), wherein the color reference card (110) comprises a plurality of different color reference fields (112) having known reference color values and one or more gray fields (114) having a defined gray value.

14. A computer program comprising instructions which, when the program is executed by a mobile device (128) having a camera (130), cause the mobile device (128) to carry out at least steps a) to d) of the methods according to any one of claims 1 to 11.

15. A computer-readable storage medium, specifically a non-transitory storage medium, comprising instructions which, when executed by a mobile device (128) having a camera (130), cause the mobile device (128) to carry out at least steps a) to e) of the method according to any one of the preceding method claims.

## Patentansprüche

1. Verfahren zum Steuern der Einstellungen des automatischen Weißabgleichs eines Mobilgerätes (128) zum Durchführen einer farbbasierten Messung unter Verwendung des Mobilgerätes (128) und einer Farbreferenzkarte (110),
- wobei das Mobilgerät (128) mindestens einen Prozessor (132), eine Farbkamera (130) und eine Vielzahl von voreingestellten Weißabgleichmodi umfasst, wobei jeder Weißabgleichmodus mit einer anderen Farbtemperatur assoziiert ist, und
- wobei die Farbreferenzkarte (110) Graufelder (114) umfasst, wobei jedes Graufeld einen bekannten speziellen Grauwert aufweist,
wobei das Verfahren Folgendes umfasst:
a. Aufnehmen eines Bildes von mindestens einem Teil der Graufelder (114) der Farbreferenzkarte (110) mit Unterstützung des mindestens einen Prozessors (132) unter Verwendung der Kamera (130) und eines der Weißabgleichmodi,
i. wobei das Bild eine Vielzahl von Pixeln und Farbinformationen für mindestens drei verschiedene Farben für die Vielzahl von Pixeln umfasst, wobei jede Farbe einer Lichtwellenlänge entspricht,
**dadurch gekennzeichnet, dass** das Verfahren ferner Folgendes umfasst:
b. Bestimmen, für eine Region von Interesse innerhalb des Bildes, einer Anzahl von Graufeldern (114), für die die Farbinformationen für eine erste Farbe Überbelichtung zeigen, indem sie eine voreingestellte Schwelle überschreiten, als eine erste Überbelichtungszahl mittels des Prozessors (132),
c. Bestimmen, für die Region von Interesse innerhalb des Bildes, einer Anzahl von Graufeldern (114), für die die Farbinformationen für eine zweite Farbe Überbelichtung zeigen, indem sie eine voreingestellte Schwelle überschreiten, als eine zweite Überbelichtungszahl mittels des Prozessors (132),
i. wobei sich die erste Farbe auf eine kleinere Wellenlänge oder einen Bereich von kleineren Wellenlängen bezieht als die zweite Farbe,
d. Neuaufnehmen des Bildes von Schritt a) mit einem mit einer wärmeren Farbtemperatur assoziierten Weißabgleichmodus mit Unterstützung des Prozessors (132), wenn eine von der ersten und zweiten Überbelichtungszahl oder eine Summe der ersten und zweiten Überbelichtungszahl eine entsprechende voreingestellte Überbelichtungsschwelle überschreitet und die erste Überbelichtungszahl größer ist als die zweite Überbelichtungszahl,
e. Neuaufnehmen des Bildes von Schritt a) mit einem mit einer kälteren Farbtemperatur assoziierten Weißabgleichmodus mit Unterstützung des Prozessors (132), wenn eine von der ersten und zweiten Überbelichtungszahl oder eine Summe der ersten und zweiten Überbelichtungszahl eine entsprechende voreingestellte Überbelichtungsschwelle überschreitet und die erste Überbelichtungszahl kleiner ist als die zweite Überbelichtungszahl.

2. Verfahren nach Anspruch 1, wobei Schritte b) und c) Folgendes umfassen:
a. Identifizieren mindestens eines Teils der Farbinformationen für die jeweilige Farbe, die den Graufeldern innerhalb der Region von Interesse entsprechen,
b. Bestimmen eines Leitwertes für den identifizierten Teil der Farbinformationen,
c. Bestimmen, ob der Leitwert eine vorbestimmte Überbelichtungsschwelle überschreitet.

3. Verfahren nach Anspruch 1 oder 2, wobei die zweite Farbe rot ist und die erste Farbe blau oder grün ist.

4. Verfahren nach einem der vorstehenden Ansprüche, wobei die farbbasierte Messung eine Analytmessung ist, die auf einer Farbbildungsreaktion basiert, wobei die Farbbildungsreaktion in einem optischen Teststreifen (118) mit einer auf ein Testfeld (120) des Teststreifens (118) aufgebrachten Probe stattfindet.

5. Verfahren nach einem der vorstehenden Ansprüche, wobei die Farbreferenzkarte (110) ferner eine Vielzahl von verschiedenen Farbreferenzfeldern (112) mit bekannten Referenzfarbwerten umfasst.

6. Verfahren nach einem der vorstehenden Ansprüche, wobei das in Schritt a) aufgenommene Bild zusätzlich mindestens einen Teil des Testfeldes (120) des optischen Teststreifens (118) aufnimmt.

7. Verfahren nach einem der vorstehenden Ansprüche, wobei das Verfahren ferner Folgendes umfasst:
a. Bestimmen einer Konzentration des Analyten in der Probe unter Verwendung des in Schritt a) aufgenommenen Bildes,
i. wenn eine Summe der ersten Überbelichtungszahl und der zweiten Überbelichtungszahl kleiner ist als die voreingestellte Gesamtüberbelichtungsschwelle und/oder
ii. wenn eine Differenz zwischen der ersten Überbelichtungszahl und der zweiten Überbelichtungszahl kleiner ist als eine voreingestellte Abgleichschwelle.

8. Verfahren nach einem der vorstehenden Ansprüche, wobei Schritte a) bis e) wiederholt werden,
a. bis eine Differenz zwischen der ersten Überbelichtungszahl und der zweiten Überbelichtungszahl kleiner ist als eine voreingestellte Abgleichschwelle oder
b. bis Schritt a) für alle Weißabgleichmodi der Vielzahl von Weißabgleichmodi durchgeführt worden ist oder
c. bis eine Differenz zwischen der ersten Überbelichtungszahl und der zweiten Überbelichtungszahl im Vergleich zu einer Differenz, die basierend auf einem früher aufgenommenen Bild bestimmt wurde, beginnt, zuzunehmen, und dann eine Konzentration des Analyten in der Probe
d. unter Verwendung des Bildes, das der kleinsten Differenz zwischen der ersten Überbelichtungszahl und der zweiten Überbelichtungszahl entspricht, oder
e. unter Verwendung eines unter Verwendung des automatischen Weißabgleichmodus aufgenommenen Zusatzbildes, das der kleinsten Differenz zwischen der ersten Überbelichtungszahl und der zweiten Überbelichtungszahl entspricht, bestimmt wird.

9. Verfahren nach einem der vorstehenden Ansprüche, wobei das Mobilgerät (128) eine Anzeige (134) umfasst und das Verfahren ferner den folgenden Schritt umfasst:
a. Bereitstellen einer visuellen Führung auf der Anzeige (134) zum Positionieren der Kamera (130) in Bezug auf ein Motiv, wobei das Motiv mindestens einen Teil des optischen Teststreifens (118) und/oder mindestens einen Teil der Farbreferenzkarte (110) umfasst.

10. Verfahren nach einem der vorstehenden Ansprüche, wobei die Farbreferenzkarte mindestens einen Positionsmarker (122) umfasst.

11. Verfahren nach Anspruch 10, wobei das Verfahren automatisch initiiert wird, wenn basierend auf dem mindestens einen Positionsmarker (122) bestimmt wird, dass sich die Kamera (130) in einer definierten Position in Bezug auf die Farbreferenzkarte (110) befindet.

12. Mobilgerät (128) mit mindestens einer Kamera (130) und mindestens einer Anzeige (134), wobei das Mobilgerät (128) dafür ausgebildet ist, das Verfahren nach einem der vorstehenden Ansprüche auszuführen.

13. Kit zum Bestimmen der Konzentration eines Analyten in einer Körperflüssigkeit, wobei der Kit Folgendes umfasst:
a. das Mobilgerät nach Anspruch 12,
b. mindestens einen optischen Teststreifen (118) mit mindestens einem Reagenztestfeld (120),
c. mindestens eine Farbreferenzkarte (110), wobei die Farbreferenzkarte (110) eine Vielzahl von verschiedenen Farbreferenzfeldern (112) mit bekannten Referenzfarbwerten und ein oder mehrere Graufeld(er) (114) mit einem definierten Grauwert umfasst.

14. Computerprogramm, das Anweisungen umfasst, die, wenn das Programm von einem Mobilgerät (128) mit einer Kamera (130) ausgeführt wird, das Mobilgerät (128) veranlassen, mindestens Schritte a) bis d) der Verfahren nach einem der Ansprüche 1 bis 11 auszuführen.

15. Computerlesbares Speichermedium, speziell ein nicht flüchtiges Speichermedium, das Anweisungen umfasst, die, wenn sie von einem Mobilgerät (128) mit einer Kamera (130) ausgeführt werden, das Mobilgerät (128) veranlassen, mindestens Schritte a) bis e) des Verfahrens nach einem der vorstehenden Verfahrensansprüche auszuführen.

## Revendications

1. Procédé permettant de contrôler les réglages automatiques de balance des blancs d'un appareil mobile (128) pour réaliser une mesure basée sur les couleurs à l'aide de l'appareil mobile (128) et d'une carte de référence des couleurs (110),
- dans lequel l'appareil mobile (128) comprend au moins un processeur (132), une caméra couleur (130) et une pluralité de modes de balance des blancs préréglés, chaque mode de balance des blancs étant associé à une température de couleur différente, et
- dans lequel la carte de référence des couleurs (110) comprend des champs des gris (114) chaque champ des gris ayant une valeur des gris dédiée connue,
le procédé comprenant :
a. la capture, supportée par l'au moins un processeur (132), à l'aide de la caméra (130) et de l'un des modes de balance des blancs d'une image d'au moins une partie des champs des gris (114) de la carte de référence des couleurs (110),
i. l'image comprenant une pluralité de pixels et des informations de couleurs pour au moins trois couleurs différentes pour la pluralité de pixels, chaque couleur correspondant à une longueur d'onde de lumière,
**caractérisé en ce que** le procédé comprend en outre :
b. la détermination, par le processeur (132), pour une région d'intérêt à l'intérieur de l'image d'un nombre de champs des gris (114) pour lesquels les informations de couleurs pour une première couleur présentent une surexposition en dépassant un seuil préréglé en tant que premier nombre de surexposition,
c. la détermination, par le processeur (132), pour la région d'intérêt à l'intérieur de l'image d'un nombre de champs des gris (114) pour lesquels les informations de couleurs pour une deuxième couleur présentent une surexposition en dépassant un seuil préréglé en tant que second nombre de surexposition
i. dans lequel la première couleur se rapporte à une longueur d'onde inférieure ou une plage de longueurs d'onde inférieures à la deuxième couleur,
d. la recapture, supportée par le processeur (132), de l'image de l'étape a) avec un mode de balance des blancs associé à une température de couleur plus chaude si un parmi les premier et second nombres de surexposition ou une somme des premier et second nombres de surexposition dépasse un seuil de surexposition préréglé respectif, et le premier nombre de surexposition est supérieur au second nombre de surexposition,
e. la recapture, supportée par le processeur (132), de l'image de l'étape a) avec un mode de balance des blancs associé à une température de couleur plus froide si un parmi les premier et second nombres de surexposition ou une somme des premier et second nombres de surexposition dépasse un seuil de surexposition préréglé respectif, et le premier nombre de surexposition est inférieur au second nombre de surexposition.

2. Procédé selon la revendication 1, dans lequel les étapes b) et c) comprennent :
a. l'identification d'au moins une partie des informations de couleurs pour la couleur respective correspondant aux champs des gris dans la région d'intérêt,
b. la détermination d'une valeur guide pour la partie identifiée des informations de couleurs
c. la détermination si la valeur guide dépasse un seuil de surexposition prédéterminé.

3. Procédé selon la revendication 1 ou 2, dans lequel la deuxième couleur est le rouge et la première couleur est le bleu ou le vert.

4. Procédé selon l'une quelconque des revendications précédentes, dans lequel la mesure basée sur les couleurs est une mesure d'analyte basée sur une réaction de formation de couleurs, dans lequel la réaction de formation de couleurs se produit dans une bandelette de test optique (118) ayant un échantillon appliqué sur un champ de test (120) de la bandelette de test (118).

5. Procédé selon l'une quelconque des revendications précédentes, dans lequel la carte de référence des couleurs (110) comprend en outre une pluralité de champs de référence de couleurs (112) différents ayant des valeurs de couleurs de référence connues.

6. Procédé selon l'une quelconque des revendications précédentes, dans lequel l'image capturée à l'étape a) capture de plus au moins une partie du champ de test (120) de la bandelette de test optique (118).

7. Procédé selon l'une quelconque des revendications précédentes, dans lequel le procédé comprend en outre :
a. la détermination d'une concentration de l'analyte dans l'échantillon à l'aide de l'image capturée à l'étape a),
i. si une somme du premier nombre de surexposition et du second nombre de surexposition est inférieure au seuil de surexposition global préréglé et/ou
ii. si une différence entre le premier nombre de surexposition et le second nombre de surexposition est inférieure à un seuil de balance préréglé.

8. Procédé selon l'une quelconque des revendications précédentes, dans lequel les étapes a) à e) sont itérées
a. jusqu'à ce qu'une différence entre le premier nombre de surexposition et le second nombre de surexposition soit inférieure à un seuil de balance préréglé, ou
b. jusqu'à ce que l'étape a) ait été réalisée pour tous les modes de balance des blancs de la pluralité des modes de balance des blancs, ou
c. jusqu'à ce qu'une différence entre le premier nombre de surexposition et le second nombre de surexposition commence à augmenter par comparaison à une différence déterminée basée sur une image précédemment capturée,
et ensuite une concentration de l'analyte dans l'échantillon est déterminée
d. à l'aide de l'image correspondant à la plus petite différence entre le premier nombre de surexposition et le second nombre de surexposition, ou
e. à l'aide d'une image supplémentaire capturée à l'aide du mode automatique de balance des blancs correspondant à la plus petite différence entre le premier nombre de surexposition et le second nombre de surexposition.

9. Procédé selon l'une quelconque des revendications précédentes, dans lequel l'appareil mobile (128) comprend un affichage (134), et le procédé comprend en outre l'étape de :
a. fourniture d'un guidage visuel sur l'affichage (134) pour positionner la caméra (130) par rapport à une scène, la scène comprenant au moins une partie de la bandelette de test optique (118) et/ou au moins une partie de la carte de référence des couleurs (110).

10. Procédé selon l'une quelconque des revendications précédentes, dans lequel la carte de référence des couleurs comprend au moins un marqueur de position (122).

11. Procédé selon la revendication 10, dans lequel le procédé est initié automatiquement dans le cas où il est déterminé que la caméra (130) est dans une position définie par rapport à la carte de référence des couleurs (110) basée sur au moins un marqueur de position (122).

12. Appareil mobile (128) ayant au moins une caméra (130) et au moins un affichage (134), l'appareil mobile (128) étant configuré pour réaliser le procédé selon l'une quelconque des revendications précédentes.

13. Kit de détermination de la concentration d'un analyte dans un fluide corporel, le kit comprenant :
a. l'appareil mobile selon la revendication 12,
b. au moins une bandelette de test optique (118) ayant au moins un champ de test de réactif (120),
c. au moins une carte de référence des couleurs (110), la carte de référence des couleurs (110) comprenant une pluralité de champs de référence de couleurs (112) différents ayant des valeurs de couleurs de référence connues et un ou plusieurs champs des gris (114) ayant une valeur des gris définie.

14. Programme informatique comprenant des instructions qui, lorsque le programme est exécuté par un appareil mobile (128) ayant une caméra (130), amènent l'appareil mobile (128) à mettre en œuvre au moins les étapes a) à d) des procédés selon l'une quelconque des revendications 1 à 11.

15. Support de stockage lisible par ordinateur, spécifiquement un support de stockage non transitoire, comprenant des instructions qui, lorsqu'elles sont exécutées par un appareil mobile (128) ayant une caméra (130), amènent l'appareil mobile (128) à mettre en œuvre au moins les étapes a) à e) du procédé selon l'une quelconque des revendications de procédé précédentes.
